(19) Europäisches Patentamt

European Patent Office

Office européen des brevets

(11) Numéro de publication : **0 345 133 B1**

(12) **FASCICULE DE BREVET EUROPEEN**

(45) Date de publication du fascicule du brevet :
02.01.92 Bulletin 92/01

(51) Int. Cl.⁵ : **F16B 5/02,** F16B 39/10, A61B 17/58, A61F 2/34

(21) Numéro de dépôt : 89401450.5

(22) Date de dépôt : 26.05.89

(54) **Dispositif de fixation d'une pièce sur un support, notamment d'un implant sur un os.**

(30) Priorité : 30.05.88 FR 8807170

(43) Date de publication de la demande :
06.12.89 Bulletin 89/49

(45) Mention de la délivrance du brevet :
02.01.92 Bulletin 92/01

(84) Etats contractants désignés :
AT BE CH DE ES FR GB GR IT LI LU NL SE

(56) Documents cités :
EP-A- 0 201 024
EP-A- 0 234 939
EP-A- 0 265 712

(56) Documents cités :
BE-A- 518 327
FR-A- 2 103 104
FR-A- 2 427 507
US-A- 2 526 959

(73) Titulaire : **Surer, Patrick**
**Rue du Prieuré-de-Béré**
**F-44110 Chateaubriant (FR)**

(72) Inventeur : **Surer, Patrick**
**Rue du Prieuré-de-Béré**
**F-44110 Chateaubriant (FR)**

(74) Mandataire : **Moncheny, Michel et al**
**c/o Cabinet Lavoix 2 Place d'Estienne d'Orves**
**F-75441 Paris Cedex 09 (FR)**

## Description

La présente invention concerne un dispositif de solidarisation d'une pièce sur un support à l'aide d'au moins un organe de fixation ancré dans le support.

Il est connu (BE-A-518327) de fixer une pièce en acier sur un support également en acier à l'aide de vis comprenant une tête et une tige filetée, cette dernière passant à travers la pièce pour se visser dans un taraudage usiné dans le support, tandis que la tête vient au contact de la pièce. Il est également connu de bloquer la vis, par exemple au moyen d'un capuchon vissé dans un logement de la pièce et en appui contre la tête de la vis.

Le vissage de la vis dans le support se traduit par un effort de compression au contact entre la pièce et le support, qui est supposé générer des forces de frottement à l'interface pièce-support qui doivent éviter tout mouvement relatif de la pièce par rapport au support.

Un tel agencement ne convient pas lorsque le matériau du support est sensiblement moins dur que celui de la pièce.

Un premier problème se pose dans le cas où la matière du support s'altère à l'interface avec la pièce car les forces de frottement diminuent jusqu'à devenir nulles, ce qui rend la pièce sensible aux efforts suivant des directions perpendiculaires à son axe longitudinal, et peut provoquer l'apparition d'un jeu de la vis dans le support, puis à la longue son arrachement, et à tout le moins laisse une certaine liberté à la pièce, intolérable dans certaines applications.

C'est le cas d'un mur en béton, revêtu d'une couche de finition de faible dureté, sur lequel on doit fixer une pièce à l'aide d'au moins une vis. La couche de finition peut s'altérer et bien que la vis soit fixée dans le béton et parfaitement ancrée, la pièce acquiert une certaine liberté de déplacement par rapport au mur, préjudiciable à la bonne tenue de l'assemblage. Sous l'effet de cycles répétés de chargement-déchargement qui conduisent à des déplacements relatifs de la pièce par rapport à la vis, celle-ci peut acquérir une certaine mobilité par dégradation progressive autour de ses filets de la matière du support, dans ce cas le béton.

Pour certaines utilisations, le problème s'avère plus important encore, notamment dans le cas de la chirurgie où des implants doivent être fixés sur des os. En cas de fracture par exemple, il est nécessaire pour obtenir un montage stable de supprimer tout mouvement relatif des fragments ainsi que celui de ces fragments par rapport à l'implant.

Par ailleurs, des phénomènes d'impaction peuvent se produire entre les fragments d'os ou entre ces fragments et l'implant, ce qui conduit à un déplacement de l'implant par rapport à la vis, si l'on considère qu'il n'y a pas déplacement de la vis par rapport à l'os dans lequel elle est vissée. Le montage est alors instable et la géométrie initiale n'est pas conservée.

Un perfectionnement d'un assemblage d'une pièce sur un support à l'aide de vis consiste à orienter les axes longitudinaux des vis de façon oblique l'un par rapport à l'autre.

Dans ce cas, la dégradation de l'interface confère une certaine liberté à la pièce qui, lorsqu'elle est soumise à des efforts, tend à exercer des couples sur les vis, puisque la tête de la vis est libre en translation et en rotation par rapport à la pièce. L'axe de la vis préalablement divergent par rapport à la direction de l'effort d'arrachement se retrouve progressivement parallèle a ce celui-ci, facilitant l'arrachement.

On connaît par ailleurs (US-A-4683108), un dispositif utilisé dans un réacteur nucléaire et permettant de bloquer et de remplacer des vis. Ce dispositif comprend une coupelle de blocage à expansion, qui est reçue dans un logement de la pièce dans lequel est également disposée la tête de la vis, et qui est solidaire en rotation de cette tête. De la sorte, la tête de vis est empêchée de tourner par la coupelle et elle est également retenue en cas de rupture accidentelle de la tige. Un tel dispositif offre cependant des inconvénients tels qu'il ne résout pas le problème évoqué ci-dessus. En effet :

— il n'assure pas une résistance axiale importante aux efforts axiaux tendant à éloigner la tête de la vis de sa surface d'appui adjacente car cette résistance est limitée à l'effort nécessaire pour déformer la paroi latérale de la coupelle ;

— de la même manière, il ne réalise pas une solidarisation efficace de la tête de vis et de la pièce contre laquelle elle est en appui car la coupelle n'exerce pas d'effort axial sur la tête de la vis ;

— il nécessite un outillage tout-à-fait spécifique pour réaliser l'expansion de la paroi latérale de la coupelle de blocage ;

— il n'est pas démontable puisque la déformation de la coupelle est permanente.

Un agencement analogue est décrit dans le document EP-A-234939.

La présente invention a pour but de proposer un dispositif de solidarisation d'une pièce et d'un support, à l'aide d'au moins un organe de fixation, qui reste stable lorsque la matière du support est susceptible de se dégrader à l'interface pièce-support et qui offre une meilleure résistance à l'arrachement. Ce dispositif doit de plus être simple, peu coûteux, facile à mettre en place et à démonter.

A cet effet, elle a pour objet un assemblage de deux éléments tels qu'une pièce et un support à l'aide d'au moins un organe de fixation comportant une tête et une tige ancrée dans le matériau du support, la pièce délimitant un passage à travers lequel passe la tige de l'organe de fixation et un lamage fileté dont le diamètre est supérieur à celui de la tête de l'organe de fixation de façon à définir un fond constituant une surface d'appui pour ladite tête, un organe supplémentaire de blocage étant reçu et vissé dans ce lamage en exerçant sur la tête de l'organe de fixation un effort axial de compression qui l'applique contre le fond du lamage, caractérisé en ce que le matériau du support ayant une dureté plus faible que celle de la pièce l'organe de fixation est ancré dans le matériau du support sans provoquer d'effort de compression notable à l'interface entre les deux éléments.

L'invention va être décrite ci-dessous, en regard des dessins annexés sur lesquels :

— la Fig. 1 représente une vue en coupe d'un dispositif selon l'invention ;

— la Fig. 2 représente un dispositif avec plaque et vis selon l'invention ;

— la Fig. 3 est une vue en perspective avec arrachement d'un dispositif selon l'invention appliqué à la fixation d'une pièce cotyloïdienne perfectionnée à cet effet ;

— la Fig. 4 est une vue éclatée d'une variante de réalisation.

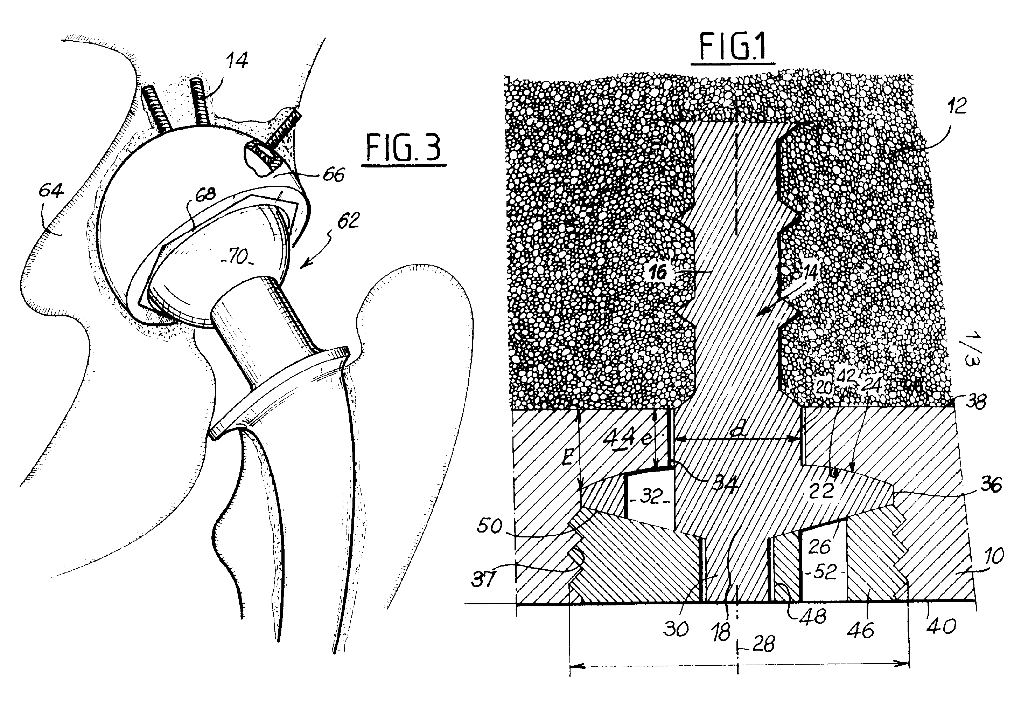

A la Fig. 1 est représenté un dispositif selon l'invention assurant la fixation d'une pièce 10 sur un support 12 à l'aide d'une vis 14.

Cette vis comprend une tige 16 filetée qui pénètre dans la matière constituant le support et une tête 18 qui vient coopérer avec la pièce 10. La tête 18 comprend une partie intermédiaire cylindrique non filetée 20 de diamètre "d", une collerette 22 d'un diamètre "D" supérieur au diamètre $\underline{d}$ dont la face 24 orientée vers la tige est convexe et dont la face 26 opposée est de préférence tronconique, ainsi qu'un pion 30 cylindrique.

Par ailleurs, dans la collerette sont prévus des alésages 32 répartis régulièrement. Dans l'exemple représenté les alésages sont au nombre de trois disposés à 120°.

La pièce 10 comprend un passage 34 et un lamage 36 débouchant respectivement sur les faces 38 dirigée vers le support, et 40 dirigée vers l'extérieur.

Le passage 34 est d'un diamètre légèrement supérieur au diamètre $\underline{d}$. Quant au lamage 36 il est coaxial avec ce passage. Il comprend en outre un taraudage 37 qui a de préférence un pas inverse de celui de la vis. Le fond 42 du lamage est concave pour coopérer par complémentarité de forme avec la face 24 de la collerette.

L'épaisseur de la zone 44 qui entoure le passage 34, comprise entre la face 38 et le fond 42 du lamage, augmente progressivement radialement vers l'extérieur de $\underline{e}$ à $\underline{E}$.

Un organe de blocage fileté 46 que l'on désignera également par le terme de vis coopère avec la partie taraudée du lamage 36. Cette vis 36 comprend un alésage 48 centré de façon à être coaxial avec la vis et adapté pour recevoir le pion 30 avec un faible jeu.

La face d'extrémité 50 de la vis 46 a un profil complémentaire de celui de la face 26 de la collerette 22.

La vis de blocage 46 est également munie d'alésages 52 analogues aux alésages 32 de la collerette et répartis de la même façon.

Les dimensions des divers éléments qui viennent d'être décrits sont liées entre elles.

Ainsi, lorsque la collerette 22 vient au contact du fond 42 du lamage 36, le pion 30 doit affleurer la face accessible 40 sans faire saillie. De même la longueur de la vis de blocage 46 doit être telle que lorsque son extrémité 50 vient en appui sur la face 26 de la collerette, cette vis affleure la face accessible 40 sans faire saillie.

La mise en place d'un tel dispositif s'effectue de la façon suivante : la vis 14 est vissée dans le support 12 jusqu'à la profondeur souhaitée, par exemple jusqu'à ce que la pièce 10 vienne au contact du support mais sans pour autant exercer une pression notable sur ce support. Cette opération est effectuée à l'aide d'un outil, non représenté, comprenant des ergots coopérant avec les alésages 32.

La vis de blocage 46 est vissée dans le lamage 36, de préférence à l'aide du même outil que précédemment jusqu'à appliquer fermement la collerette 22 contre le fond 24 du lamage, assurant par conséquent la solidarisation de la vis 14 par rapport à la pièce 10.

Du fait de cette solidarisation efficace, les efforts axiaux exercés sur la pièce sont transmis au matériau du support par l'intermédiaire des filets de la vis 14, sans intervention de l'interface entre la pièce et le support. Contrairement aux agencements connus, la solidarisation de la pièce 10 et du support 12 ne dépend pas d'une force de compression qui les applique l'un contre l'autre, ni par conséquent des forces de frottement au niveau de leur interface, mais résulte d'un effet de scellement réalisé par l'ancrage de l'organe de fixation (vis 14 ou autre) dans le support, puis de la fixation de la pièce 10 sur cet organe au moyen de la vis 46.

Les efforts transversaux sont repris par la partie intermédiaire 20 et le pion 30 qui, respectivement en appui sur les parois du passage 34 et sur les parois de l'alésage 48 de la vis de blocage 46, s'opposent à la rotation de la vis par rapport à la plaque, engendrée par de telles composantes.

En variante, la vis de fixation est vissée dans le support sans que la pièce vienne directement au contact de ce dernier. Cela peut correspondre au cas où une couche d'un matériau de faible dureté (non représentée) est interposée entre le support 12 et la pièce 10.

Selon une autre variante la vis 14 peut être remplacée par un clou ou autre organe de fixation non fileté et comportant simplement une tige ancrée dans le matériau du support 12.

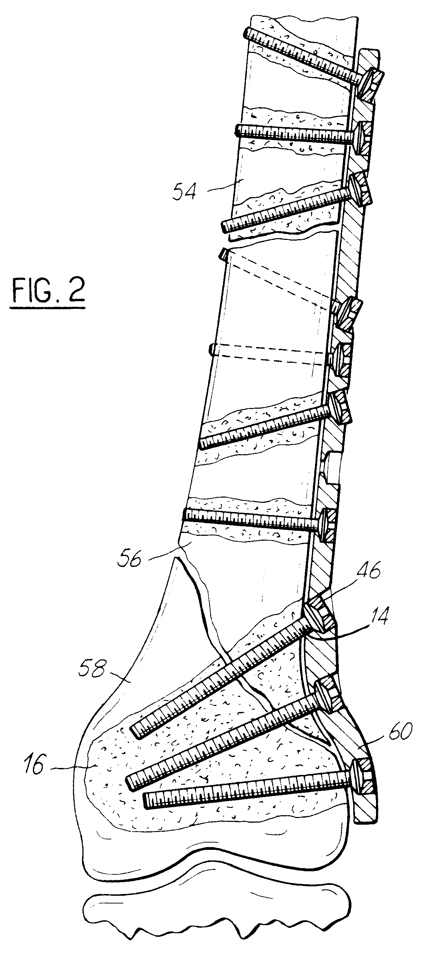

La Fig. 2 représente une application de l'invention dans le domaine médical, où le support 12 de la Fig. 1 est constitué par un fémur 54 ayant subi une double fracture de l'épiphyse et de la diaphyse. La pièce 10 est alors une plaque 60 qui maintient en position les deux fragments 56 et 58.

Cette plaque comprend plusieurs vis de fixation du type décrit ci-dessus, dont la tige passe à travers la plaque et dont la tête est fixée dans la plaque par des vis de blocage correspondantes.

Dans cette application, le matériau du support comprend l'os formé par la matière osseuse corticale proprement dite et l'os spongieux de faible résistance mécanique disposé dans la partie centrale de l'os.

Les logements des vis de fixation, c'est-à-dire les passages et les lamages, sont orientés de façon que les axes des vis soient orientés obliquement les uns par rapport aux autres, ce qui augmente la résistance à l'arrachement.

De même, pour améliorer la tenue des vis de fixation, celles-ci sont généralement traversantes pour coopérer avec la partie de l'os la plus résistante mécaniquement, c'est-à-dire la matière osseuse située à l'opposé de la plaque.

Sur cette Fig. 2, le fragment 58 est maintenu en position par rapport à la plaque y compris dans le cas d'une dégradation de la matière osseuse à l'interface avec la plaque. En effet, les vis sont immobilisées par rapport au fragment 58 et, comme les vis sont elles-mêmes immobilisées par rapport à la plaque, le fragment 58 conserve sa position initiale tant en translation qu'en rotation autour d'une des têtes de vis.

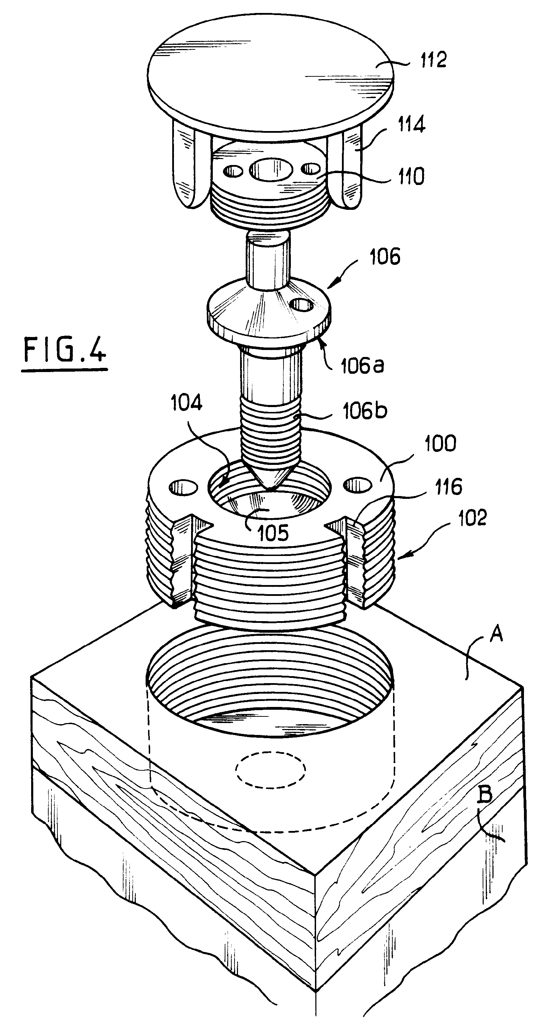

La Fig. 3 représente une autre application de l'invention dans le domaine médical, à savoir la fixation d'une pièce cotyloïdienne 62 sur l'os iliaque 64 dans le cas d'une prothèse de hanche. La coquille 66 de la pièce cotyloïdienne 62 est sensiblement hémisphérique et comporte des logements répartis sur sa surface destinés à recevoir des vis de fixation 14 et de blocage 46 telles que décrites ci-dessus.

La forme intérieure de la coquille de la pièce cotyloïdienne est adaptée pour recevoir une chemise 68, masquant les logements et les têtes de vis, qui est destinée à coopérer avec une rotule 70 d'une partie complémentaire de la pièce cotyloïdienne.

Cette chemise 68 doit être immobilisée en rotation par rapport à la coquille et, dans ce but, elles ont toutes deux, respectivement extérieurement et intérieurement, la forme d'un tronc de pyramide.

Dans ce cas, lorsque la pièce cotyloïdienne est soumise à des efforts de compression, ceux-ci sont repris par les vis de blocage qui suppriment tout mouvement de la pièce cotyloïdienne par rapport aux vis de fixation et donc tout mouvement de la pièce cotyloïdienne par rapport à l'os puisque les vis de fixation sont ancrées dans la matière osseuse.

Il est possible d'interposer, si cela s'avère nécessaire, de la matière pour combler les cavités qui résultent du fait que le cotyle dans lequel vient se loger la pièce cotyloïdienne peut être détérioré. Cette matière ne subit pas d'efforts de compression ni de cisaillement puisque ceux-ci sont reportés par les vis directement dans la matière osseuse de l'os iliaque.

Les ancrages que constituent les vis peuvent ainsi être reportés à une distance relativement importante de la pièce.

Dans le domaine médical, le dispositif selon l'invention n'est pas limité aux seules plaques et pièces cotyloïdiennes mais s'applique à la fixation de tous les types d'implants ou de matériel ancillaire, c'est-à-dire d'un matériel fixé de façon temporaire et qui permet de positionner une prothèse.

Dans l'exemple de la Fig. 4, l'invention est adaptée au cas d'une pièce A non métallique et dont le matériau se prête mal à la réalisation d'un chambrage taraudé de diamètre relativement faible. Pour résoudre cette difficulté, il est prévu une pièce rapportée métallique 100 comportant un filetage externe 102 et un chambrage muni d'un filetage interne 104. Cette pièce rapportée délimite une surface d'appui 105 pour la tête 106a d'un organe de fixation 106 et un passage pour la tige 106b de cet organe de fixation. Le dispositif est complété par un organe de blocage 110 fileté extérieurement ainsi que par un chapeau 112 muni de pattes 114 qui viennent s'engager dans des rainures 116 ménagées à la périphérie de la pièce 100.

L'utilisation d'un tel dispositif est la suivante : on perce au moyen d'un foret un avant-trou dans les deux éléments A, B à assembler, puis au moyen d'un foret creux on ménage dans l'élément A un logement destiné à recevoir la pièce 100. On visse cette dernière dans ce logement, puis on fixe l'organe de fixation dans l'élément B, on visse l'organe de blocage dans la pièce 100 et enfin on met en place le chapeau 112 qui va assurer le blocage de la pièce rapportée par rapport à l'élément A.

Bien que l'invention trouve une application particulièrement intéressante dans le domaine chirurgical, elle peut également présenter de grands avantages dans d'autres secteurs, tels que le bâtiment ou le bricolage et y remplacer les pattes-fiches ou pattes de scellement qui nécessitent l'utilisation de plâtre, de ciment ou autre matériau de scellement et n'offrent pas les mêmes possibilités, en particulier de démontage.

## Revendications

1. Assemblage de deux éléments tels qu'une pièce (10) et un support (12) à l'aide d'au moins un organe de fixation (14) comportant une tête (18) et une tige (16) ancrée dans le matériau du support, la pièce (10) délimitant un passage (34) à travers lequel passe la tige (16) de l'organe de fixation et un lamage fileté (36) dont le diamètre est supérieur à celui de la tête (18) de l'organe de fixation de façon à définir un fond (42) constituant une surface d'appui pour ladite tête, un organe supplémentaire de blocage étant reçu et vissé dans ce lamage en exerçant sur la tête (18) de l'organe de fixation un effort axial de compression qui l'applique contre le fond (42) du lamage, caractérisé en ce que le matériau du support ayant une dureté plus faible que celle de la pièce (10), l'organe de fixation (14) est ancré dans le matériau du support sans provoquer d'effort de compression notable à l'interface entre les deux éléments (10, 12 ; A, B).

2. Assemblage selon la revendication 1, caractérisé en ce que l'organe de fixation (14) est une vis.

3. Assemblage selon la revendication 2, caractérisé en ce que le lamage (36) est taraudé avec un pas inverse à celui du filetage de la tige (16) de la vis de fixation (14).

4. Assemblage selon la revendication 1, caractérisé en ce que l'organe de fixation (14) est un clou.

5. Assemblage selon l'une quelconque des revendications précédentes, caractérisé en ce que la tête (18) de l'organe de fixation (14) et la vis de blocage (46) comprennent des moyens complémentaires de maintien latéral.

6. Assemblage selon la revendication 5, caractérisé en ce que les moyens complémentaires de maintien latéral comprennent un pion (30) en saillie sur la tête de l'organe de fixation (14) et un logement central (48) ménagé dans la vis de blocage (46).

7. Assemblage selon la revendication 2, caractérisé en ce que la tête (18) de la vis de fixation (14) et la vis de blocage (46) comprennent des évidements (32, 52) excentrés destinés à coopérer avec un outil de manoeuvre.

8. Assemblage selon l'une quelconque des revendications 1 à 7, caractérisé en ce que la pièce (10) a une épaisseur progressivement croissante à partir de la zone délimitant le passage (34).

9. Assemblage selon l'une quelconque des revendications précédentes, caractérisé en ce qu'il comprend une pièce rapportée (100) comportant un filetage externe (102) et vissée dans le matériau de la pièce (A), cette pièce rapportée délimitant une surface d'appui (105) pour la tête (106a) de l'organe de fixation (106), un passage pour la tige (106b) de cet organe de fixation et un lamage muni d'un filetage interne (104) coopérant avec l'organe supplémentaire de blocage (110).

10. Assemblage selon la revendication 9, caractérisé en ce que la pièce rapportée (100) comporte à sa périphérie au moins une rainure longitudinale (116) et le dispositif est complété par un chapeau (112) muni d'au moins une patte (114) adaptée pour venir s'engager dans la rainure (116) et bloquer la pièce rapportée en rotation par rapport à l'élément (A) dans lequel elle est vissée.

11. Assemblage suivant l'une quelconque des revendications 1 à 10, caractérisé en ce qu'il comprend au moins deux organes de fixation agencés en oblique l'un par rapport à l'autre.

12. Assemblage selon l'une quelconque des revendications 1 à 11, caractérisé en ce que le support est constitué par au moins un os et la pièce est un implant ou un élément de matériel ancillaire.

13. Assemblage selon la revendication 12, caractérisé en ce que l'implant est une pièce cotyloïdienne (62), comprenant une coquille (66) délimitant une surface externe sphérique et une surface interne de forme pyramidale tronquée, ainsi qu'une chemise (68) de forme pyramidale tronquée complémentaire délimitant un logement pour une rotule (70), des organes de fixation et des vis de blocage étant agencés dans au moins certaines des faces de la surface interne de cette coquille.

## Patentansprüche

1. Verbindung zweier Elemente, wie beispielsweise eines Teils (10) und eines Trägers (12) mit Hilfe wenigstens eines Befestigungsorgans (14), das einen Kopf (18) und einen Schaft (16) aufweist, der in dem Material des Trägers verankert ist, wobei das Teil (10) einen Durchgang (34), durch den der Schaft (16) des Befestigungsorgans verläuft, und einen Gewindegang (36) begrenzt, dessen Durchmesser größer als der des Kopfes (18) des Befestigungsorgans ist, um einen Boden (42) auszubilden, der eine Anlagefläche für den Kopf bildet, wobei ein zusätzliches Klemmorgan in diesem Gewindegang eingeschraubt ist, das auf den Kopf (18) des Befestigungsorgans eine axiale Druckkraft ausübt, die ihn gegen den Boden (42) des Gewindegangs drückt, **dadurch gekennzeichnet**, daß das Material des Trägers eine Festigkeit aufweist, die geringer als die des Teils (10) ist, das Befestigungsorgan (14) in dem Material des Trägers verankert ist, ohne eine an der Grenzfläche zwischen den zwei Elementen (10, 12 ; A, B) wahrnehmbare Druckkraft hervorzurufen.

2. Verbindung nach Anspruch 1, **dadurch gekennzeichnet**, daß das Befestigungsorgan (14) eine Schraube ist.

3. Verbindung nach Anspruch 2, **dadurch gekennzeichnet**, daß der Gewindegang (36) mit einer Steigung geschnitten ist, die umgekehrt zum Gewinde des Schaftes (16) der Befestigungsschraube (14) ist.

4. Verbindung nach Anspruch 1, **dadurch gekennzeichnet**, daß das Befestigungsorgan (14) ein Nagel ist.

5. Verbindung nach einem der vorhergehenden Ansprüche, **dadurch gekennzeichnet**, daß der Kopf (18) des Befestigungsorgans (14) und die Klemmschraube (46) komplementäre Mittel zur seitlichen Abstützung aufweisen.

6. Verbindung nach Anspruch 5, **dadurch gekennzeichnet**, daß die komplementären Mittel zur seitlichen Abstützung einen über dem Kopf des Befestigungsorgans (14) vorstehenden Zapfen und eine zentrale Aufnahme (48) in der Klemmschraube (46) umfassen.

7. Verbindung nach Anspruch 2, **dadurch gekennzeichnet**, daß der Kopf (18) der Befestigungsschraube (14) und die Klemmschraube (46) exzentrische Ausnehmungen (32, 52) aufweisen, die zum Zusammenwirken mit einem Montagewerkzeug bestimmt sind.

8. Verbindung nach einem der Ansprüche 1 bis 7, **dadurch gekennzeichnet**, daß das Teil (10) eine Dicke aufweist, die von der den Durchgang (34) begrenzenden Zone ausgehend progressiv zunimmt.

9. Verbindung nach einem der vorhergehenden Ansprüche, **dadurch gekennzeichnet**, daß es ein zusammengesetztes Teil (100) enthält, das ein Außengewinde (102) aufweist und in das Material des Teils (A) eingeschraubt ist, wobei das zusammengesetzte Teil eine Anlagefläche (105) für den Kopf (106a) des Befestigungsorgans (106) ausbildet, wobei ein Durchgang für den Schaft (106b) dieses Befestigungsorgans und ein mit einem Innengewinde (104) versehenes Gewindestück mit dem zusätzlichen Klemmorgan (110) zusammenwirkt.

10. Verbindung nach Anspruch 9, **dadurch gekennzeichnet**, daß das zusammengesetzte Teil (100) an seinem Umfang wenigstens eine Längsrille (116) aufweist und die Vorrichtung durch einen Hut (112) vervollständigt wird, der mit wenigstens einer Nase (114) versehen ist, die zum Eingreifen in die Rille (116) und zur Drehsicherung des zusammengesetzten Teils gegenüber dem Element (A), in das es eingeschraubt ist, dient.

11. Verbindung nach einem der Ansprüche 1 bis 10, **dadurch gekennzeichnet**, daß sie wenigstens zwei Befestigungsorgane enthält, die schräg zueinander angeordnet sind.

12. Verbindung nach einem der Ansprüche 1 bis 11, **dadurch gekennzeichnet**, daß der Träger von wenigstens einem Knochen gebildet ist und das Teil ein Implantat oder ein Element aus einem Hilfsmaterial ist.

13. Verbindung nach Anspruch 2, **dadurch gekennzeichnet**, daß das Implantat ein Kugelgelenk (62) ist, enthaltend eine Pfanne (66) mit einer sphärischen Außenseite und einer pyramidenstumpfförmigen Seite und mit einer Auskleidung (68) von komplementär pyramidenstumpfförmiger Gestalt, die eine Aufnahme für einen Kugelkopf (70) bildet, wobei Befestigungsorgane und Klemmschrauben in wenigstens in einigen der Flächen der Innenseite dieser Pfanne angeordnet sind.

## Claims

1. Assembly of two elements such as a part (10) and a support (12) with the aid of at least one fixation member (14) comprising a head (18) and a rod (16) anchored in the support substance, the part (10) delimiting a passage (34) through which passes the rod (16) of the fixation member and a threaded countersink (36) whose diameter is greater than that of the head (18) of the fixation member in such a way as to define a base (42) constituting a support surface for the said head, a supplementary locking member being received and screwed in this countersink by exerting on the head (18) of the fixation member an axial compression force which applies it against the base (42) of the countersink, characterised in that the support substance having a hardness lower than that of the part (10), the fixation member (14) is anchored in the support substance without causing any appreciable compression force at the interface between the two elements (10, 12 ; A, B).

2. Assembly according to Claim 1, characterised in that the fixation member (14) is a screw.

3. Assembly according to Claim 2, characterised in that the countersink (36) is tapped with a pitch which is the opposite of that of the thread of the rod (16) of the fixation screw (14).

4. Assembly according to Claim 1, characterised in that the fixation member (14) is a nail.

5. Assembly according to any one of the preceding claims, characterised in that the head (18) of the fixation member (14) and the locking screw (46) comprise complementary means for lateral holding.

6. Assembly according to Claim 5, characterised in that the complementary means for lateral holding comprise a pin (30) protruding from the head of the fixation member (14) and a central hole (48) formed in the locking screw (46).

7. Assembly according to Claim 2, characterised in that the head (18) of the fixation screw (14) and the locking screw (46) comprise eccentric recesses (32, 52) intended to cooperate with an assembly tool.

8. Assembly according to any one of Claims 1 to 7, characterised in that the part (10) has a thickness progressively increasing from the zone delimiting the passage (34).

9. Assembly according to any one of the preceding claims, characterised in that it comprises a gusset (100) comprising an external thread (102) and screwed into the material of the part (A), this gusset delimiting a support surface (105) for the head (106a) of the fixation member (106), a passage for the rod (106b) of this fixation member and a countersink provided with an internal thread (104) cooperating with the supplementary locking member (110).

10. Assembly according to Claim 9, characterised in that the gusset (100) comprises, at its periphery, at least one longitudinal groove (116) and the device is completed by a cap (112) provided with at least one tab (114) adapted so as to engage in the groove (116) and to lock the gusset in rotation relative to the element (A) in which it is screwed.

11. Assembly according to any one of Claims 1 to 10, characterised in that it comprises at least two fixation members arranged obliquely relative to each other.

12. Assembly according to any one of Claims 1 to 11, characterised in that the support consists of at least one bone and the part is an implant or an element of ancillary material.

13. Assembly according to Claim 12, characterised in that the implant is a cotyloid part (62) which consists of a shell (66) delimiting a spherical external surface and an internal surface of truncated pyramid shape, and also a jacket (68) of complementary truncated pyramid shape delimiting a housing for a ball (70), fixation members and locking screws being arranged in at least some of the faces of the internal surface of this shell.

FIG. 3

FIG.1

EP 0 345 133 B1

1/3

FIG. 2

54

56

58

16

46

14

60

FIG.4